# EUROPEAN PATENT SPECIFICATION

(11) **EP 1 104 777 B1**
(45) Date of publication and mention of the grant of the patent: **28.02.2007**
(21) Application number: 99309645.2
(22) Date of filing: 01.12.1999
(51) Int. Cl.: C08H 1/06, A61K 47/42

(54) **Use of pepsin-treated gelatin as a stabiliser for injectable trace protein substances**
Verwendung von mit Pepsin behandelter Gelatine als Stabilisator für einspritzbare Stoffe enthaltend Proteinenspuren
Utilisation de gélatine traitée par de la pepsine comme agent stabilisant pour substances injectables contenant des protéines à l'état de traces

(43) Date of publication of application: 06.06.2001
(73) Proprietor: NIPPI, INCORPORATED, Adachi-ku, Tokyo120 (JP)
(72) Inventor: Ebihara, Tetsuya, c/o Nippi Res.Inst. of Biomatrix, Adachi-ku, Tokyo (JP); Hattori, Shunji, c/o Nippi Res.Inst. of Biomatrix, Adachi-ku, Tokyo (JP); Matsubara, Youco, c/o Nippi Res.Inst. of Biomatrix, Adachi-ku, Tokyo (JP); Irie, Shinkichi, c/o Nippi Res.Inst. of Biomatrix, Adachi-ku, Tokyo (JP)
(74) Representative: Gaunt, Robert John

(56) References cited:
- DATABASE WPI Week 200010 Derwent Publications Ltd., London, GB; AN 2000-111667 XP002137413 "Novel gelatin - for stabilizing micro quantity protein composiition such as vaccines, pharmaceuticals, foodstuffs and cosmetics" & JP 11 349599 A (NIPPI KK), 21 December 1999 (1999-12-21)
- W. M. BELIKOW ET AL.: "Zwei Stadien bei der Verdaaung von Eiweissen durch Pepsin" DIE NAHRUNG, vol. 20, no. 6, 1976, pages 593-596, XP000905382 de

## Description

This invention relates to hydrolyzed gelatin, more particularly to pepsin-decomposed gelatin that is useful as a stabilizer for injectable trace protein substances such as vaccines.

Effective trace protein substances such as injectable vaccine preparations and erythropoietin contain gelatin as a stabilizer in the form of an extract from bovine or swine tissues. Gelatin is effective not only as a stabilizer of effective trace protein substances but also for preventing the coagulation of proteins in the preparations and the adsorption of the effective trace protein substances on glass surfaces and other wall surfaces of containers.

Gelatin is the extract with hot water of collagen which is abundant in connective tissues in the living body and it generally has a smaller molecular weight than collagen. Collagen generally has a high degree of homology in amino acid sequence among animal species, at least 90% in bovine, swine and humans. Thus, gelatin derived from collagen has only small allergenic action and has long been used as a stabilizer of injections.

Cases, though very few, have recently been reported of the occurrence of allergic reaction in infants after inoculation of vaccines and it has been shown that the allergen is the gelatin incorporated in vaccine preparations. Since an anti-gelatin immunoglobulin E antibody (IgE) was detected in the sera of allergic patients, it has been shown that the allergy to gelatin is of a type I allergic reaction. Human serum albumin is known as a protein having the same effect as gelatin but considering the potential risk of contamination by AIDS and hepatitis viruses, the established practice of using gelatin is believed to be on the safer side. Gelatin is also preferred from an economic viewpoint since it is available at a fairly low cost.

A report has been made in Unexamined Published Japanese Patent Application (kokai) No. 82299/1995 about less allergenic gelatin. That is, when a starting material containing a collagen component or a gelatin component which is a modified product thereof was specifically decomposed with a bacterial collagenase, there was obtained a peptide composition with a molecular weight of no more than 1,000 that had no antigenicity and which contained at least 70% of peptides having the amino acid sequence (Gly-X-Y)n (n = 1 - 3). The gelatin thus prepared by the collagenase-dependent method may be effective in food but having a molecular weight of no more than 1,000, it certainly is not suitable as a vaccine stabilizer.

It is reported in Unexamined Published Japanese Patent Application (kokai) No. 229932/1997 that a human who was allergic to gelatin was sensitive to the α2 chain in bovine-derived gelatin and that gelatins derived from other animals could be used with vaccines. However, the patent teaches nothing about preparing less allergenic gelatin from bovine-derived gelatins in general.

At a general conference held by the Japanese Society for Immunology, the present inventors read a detailed paper to show that patients manifesting an allergic reaction to gelatin were sensitive to the α2 chain in collagen or gelatin (the 27th Annual Meeting of the Japanese Society for Immunology, October 29 - 31, 1997).

Therefore, a strong need still exists for a gelatin that is useful as a stabilizer of vaccines that can safely be administered to patients who manifest an allergic reaction to gelatin.

The present invention has been accomplished under these circumstances and has the object of providing a less allergenic hydrolyzed gelatin that is useful as a stabilizer for injectable trace protein substances such as vaccines.

In order to attain the stated object, the present inventors made intensive studies and found that a gelatin hydrolyzate, or gelatin decomposed with pepsin, had a smaller degree of allergenicity. The present invention has been accomplished on the basis of this finding.

Thus, the present invention relates to the use of a pepsin-treated low allergenic gelatin as a stabilizer for injectable trace protein substances wherein said gelatin has a weight average molecular weight of 3,500 - 20,000 as measured by gel permeation high-performance liquid chromatography and has a separation pattern having characteristic bands at 15 kd, 17 kd, 36 kd, and 60 kd in SDS-polyacrylamide gel electrophoresis.

The present invention also provides an injection comprising an injectable trace protein substance, a stabilizer comprising a pepsin-treated low allergenic gelatin having a weight average molecular weight of 3,500-20,000 as measured by gel permeation high-performance liquid chromatography and having a separation pattern having characteristic bands at 15 kd, 17 kd, 36 kd, and 60 kd in SDS-polyacrylamide gel electrophoresis, and a pharmaceutically acceptable vehicle. Typically, said injectable trace protein substance comprises a vaccine and said vehicle comprises water.
Fig. 1 shows by bands the positions of hydrophobic amino acids (e.g. valine, leucine, isoleucine, methionine, phenylalanine and tryptophan) in the sequences of the α1 and α2 chains in bovine collagen;
Fig. 2 shows the separation pattern of undecomposed gelatin (thermally denatured SCE) on high-performance liquid chromatography;
Fig. 3 shows the separation pattern of pepsin-decomposed gelatin on high-performance liquid chromatography;
Fig. 4 is a photograph showing the result of SDS-polyacrylamide electrophoresis performed on gelatin after it was decomposed with various enzymes, with its reactivity with IgE being indicated qualitatively under respective bands; and
Figs. 5a and 5b are photographs showing the results of 10% SDS-polyacrylamide gel electrophoresis and 15% SDS-polyacrylamide gel electrophoresis that were respectively performed on non-antigenic collagen α1 chain and antigenic collagen α2 chain which had been separated by a CM-cellulose column and later decomposed with pepsin.

The invention is described below in greater detail.

For sensible development of less allergenic gelatin that finds a wide range of applications, it would be necessary to use the sera of as many patients as possible who are allergenic to gelatin and identify the epitope in the gelatin for serum IgE. The present inventors therefore attempted to identify the epitope for IgE in patients having an allergy to gelatin and disrupt the IgE reactive site in gelatin with a specific enzyme, thereby providing a gelatin hydrolyzate that had low allergenicity and which was highly capable of stabilizing injectable trace proteins such as vaccines.

To begin with, the present inventors collected the sera of patients showing an allergic reaction to gelatin and employed a fluorescent ELISA method to evaluate the sensitivity of IgE to gelatin and its hydrolyzate that were prepared by various known methods. The allergenicity of gelatin can be evaluated by knowing how the serum IgE of the patients would react to gelatin. Collagen is inherently composed of two different polypeptides, α1 and α2 chains, having a molecular weight of 100,000; hence, gelatin is a mixture of these two collagen-derived polypeptides. As is shown in Table 1 below, the sera of three patients reacted to the α2 chain purified from collagen but not to the purified α1 chain (details of this different behavior were reported in the Abstracts of the papers read at the 27th Annual Meeting of the Japanese Society for Immunology, supra).

**Table 1**

| | (Numerals indicate the intensity of fluorescence) | | |
|---|---|---|---|
| Antigen | Serum case 1 | Serum case 2 | Serum case 3 |
| Bovine gelatin | 2200 | 1430 | 4380 |
| Alpha 1 chain | 144 | 307 | 137 |
| Alpha 2 chain | 2500 | 1500 | 4250 |
| No antigen | 20 | 22 | 20 |

Of the two polypeptides in bovine collagen used as a source of gelatin, the α2 chain has antigenicity and its complete amino acid sequence was recently identified by the present inventors on the basis of its cDNA sequence (see T. Sirai et al., "The complete cDNA coding sequence for the bovine proα2(I) chain of type I Procollagen", Matrix Biol. 1998; DNA database EMBL/Gen-Bank AB008683).

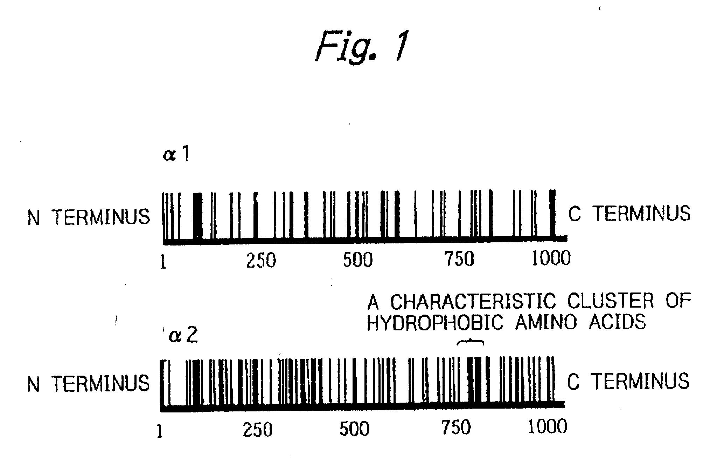

Gelatin is a protein with very small contents of hydrophobic amino acids. However, a comparison between the recently identified amino acid sequence of the α2 chain and the already known sequence of the α1 chain showed that the former contained about twice as many hydrophobic amino acid residues (62 out of the 1014 residues were occupied by the α1 chain and 104 by the α2 chain). It was also revealed that the α2 chain had a characteristic cluster of hydrophobic amino acids near the N and C terminals (see Fig. 1). A peptide fragment of the α2 chain was separated, purified and tested for reactivity with the IgE of patients; antigenicity was found in the peptides near site No. 750 in the C terminal amino acid sequence of the α2 chain, obviously in agreement with the position of the cluster of hydrophobic amino acids near the C terminal.

These findings led the present inventors to conclude that a less allergenic gelatin hydrolyzate would be obtained by disrupting the antigenic site on the α2 chain; the inventors therefore tested a variety of gelatin decomposing enzymes.

Surprisingly enough, only the gelatin that was decomposed with pepsin among various decomposing enzymes was found to be optimal for use as a stabilizer of injectable trace proteins such as vaccines. The present invention has been accomplished on the basis of this finding. Stated specifically those gelatin samples which were treated with pepsin, collagenase (both C. histricum and one derived from the crab pancreas), chymotrypsin, elastase, bromelain, ficin and alkali protease were reduced in reactivity with the IgE of allergic patients. However, the gelatin samples treated with non-pepsin enzymes, namely, collagenase (both C. histricum and one derived from the crab pancreas), chymotrypsin, elastase, bromelain, ficin and alkali protease, had lower molecular weights than 3,500 which was necessary to stabilize trace proteins and, hence, were unsuitable for the purpose of stabilizing trace proteins. In contrast, the gelatin samples that were treated with pepsin had an average molecular weight of 7,600 well in excess of 3,500 which was the required value for stabilizing trace proteins. Other enzymes could not achieve satisfactory lowering of the reactivity with IgE regardless of the molecular weight of the treated gelatin. If low-molecular weight peptides are removed by fractionation by gel permeation chromatography or microfiltration, one can obtain less allergenic peptides of higher weight average molecular weights, say 20,000, according to the invention.

The gelatin that can be used as a starting material for preparing the gelatin hydrolyzate of the invention is not limited in any way and either the acid process gelatin or the alkali process gelatin will do. Also usable is gelatin resulting from thermal denaturation of solubilized collagen. The solubilized collagen may be the solubilized collagen with acetic acid (S.C.A) known in the art or collagen solubilized with an organism-produced protease ("PROCTASE" sold by Meiji Seika Kaisha, Ltd.) or an animal-derived protease such as pepsin.

Any commercially available pepsin may be used to decompose gelatin and an example is one derived from the mucous membrane of swine stomach that is commercially available from Sigma.

For decomposing gelatin with pepsin, the following conditions are preferred. The concentration of pepsin relative to gelatin is not critical and the skilled artisan can easily choose a suitable value from common the range of protease concentrations that are sufficient to decompose proteins. For example, the weight ratio of substrate to enzyme ranges from 10,000 : 1 to 10 : 1, preferably from 200 : 1 to 50 : 1, more preferably 100 : 1.

Gelatin is preferably dissolved in 5 - 500 mmol of acetic acid or hydrochloric acid. with the gelatin concentration preferably ranging from 0.01 to several tens of percent. The decomposition temperature ranges preferably from 37°C to 60°C and the decomposition time ranges preferably from several hours to several days until the α chain with a molecular weight of 100,000 is no longer detected on electrophoresis. Any fraction of the α chain with a molecular weight of 100,000 that remains undecomposed can be rejected by gel permeation chromatography and ultrafiltration.

The pepsin used to decompose gelatin can be removed by treatment with an ion-exchange resin or a resin having an anti-pepsin antibody immobilized. If desired, resin immobilized pepsin may be used to decompose gelatin so as to avoid pepsin contamination.

The thus obtained gelatin hydrolyzate has a weight average molecular weight of 3,500 - 20,000, preferably 4,000 - 10,000, more preferably about 7,600, as measured by gel permeation high-performance liquid chromatography. It also exhibits a separation pattern having characteristic bands at 15 kd, 17 kd, 36 kd and 60 kd in SDS-polyacrylamide electrophoresis and no component is detected that derives from the collagen α2 chain.

The skilled artisan can easily determine how much of the gelatin hydrolyzate should be used as stabilizer of injectable trace protein substances.

The following examples are provided for the purpose of further illustrating the present invention but are in no way to be taken as limiting.

### Example

Type I bovine collagen (trade name: SCE) solubilized by treatment with Aspergillus produced protease (available from Meiji Seika Kaisha, Ltd. under the trade name "PROCTASE") was thermally denatured by treatment at 100°C for 2 minutes to produce gelatin, which was decomposed with various enzymes, i.e., pepsin, bacterial collagenase (C. histricum), collagenase (from the crab pancreas), chymotrypsin, elastase, bromelain, ficin, alkali protease, actinase. V8 protease, bacterial collagenase (Vibrio sp.), trypsin and papain. The pepsin was purchased from Sigma as a product derived from the mucous membrane of swine stomach and the other enzymes were purchased from either Sigma or Wako Pure Chemical Industries, Ltd. The decomposition conditions were as follows; by preliminary treatment at 100°C for 2 minutes, SCE was thermally denatured to give gelatin; to 0.1% of the gelatin, (in 0.5 M acetic acid solution), pepsin was added to give a final concentration of 0.001%; the solution was then left to stand at 37°C for 24 hours. For the other enzymes, solvents known to be optimal in the art were used; the other decomposition conditions were the same as set for above.

Assay of IgE antibody against gelatin: Enzyme-treated gelatin samples were adsorbed on an ELISA plate and the titre of IgE antibody in the sera of patients that reacted with each gelatin sample was measured. As a control, the IgE antibody against the untreated gelatin was assayed.

Measurement of average molecular weight: The average molecular weights of the enzyme-treated gelatin samples were measured by high-performance liquid chromatography. The separating column consisted of Shodex OHpak SB803 connected in series to Shodex OHpack SB802.5 (both being produced by Showa Denko K.K.). The solvent was a buffer solution of calcium phosphate (pH 6.9); the flow rate was 1 mL/min; the column temperature was 40°C. The molecular weight marker for polyethylene oxide was used as the standard. As in the case of assaying the IgE antibody against gelatin, the molecular weight of the untreated gelatin was also measured.

Table 2 shows the reactivities of the enzyme-treated gelatin samples with the IgE in the sera of patients who were allergic to gelatin.

**Table 2. Reactivity of Enzyme-Decomposed Gelatin with IgE in the Sera of Patients Having Allergy to Gelatin**

| Enzyme | Average molecular weight | Reactivity with IgE | | | |
|---|---|---|---|---|---|
| | | Patient 1 | Patient 2 | Patient 3 | Patient 4 |
| Gelatin (not treated with enzyme) | 165,617 Da | ++++ | ++++ | ++++ | ++++ |
| Pepsin | 7,666 Da | - | + | - | + |
| Bacterial collagenase (C. histricum) | 1,635 Da | - | - | - | - |
| Collagenase (crab pancreas) | 2,773 Da | - | - | - | - |
| Chymotrypsin | 3,071 Da | - | - | - | - |
| Elastase | 3,794 Da | - | - | - | - |
| Bromelain | 2,422 Da | - | - | - | - |
| Ficin | 2,055 Da | - | - | - | - |
| Alkali protease | 2,971 Da | - | - | - | - |
| Actinase | 4,113 Da | ++++ | ++++ | ++ | ++++ |
| V8 protease | 10,203 Da | +++ | ++++ | +++ | +++ |
| Bacterial collagenase (Vibrio sp.) | 6,571 Da | ++++ | ++++ | ++++ | ++++ |
| Trypsin | 2.551 Da | - | - | +++ | - |
| Papain | 4,068 Da | ++ | +++ | - | - ++++ |

| | | | | | |
|---|---|---|---|---|---|
| Reactivity with IgE is expressed as the percentage of the reactivity of gelatin (untreated) with the sera of patients as measured by ELISA and the rating is as follows: ++++, 75 - 100%; +++, 50 - 75%; ++, 25 - 50%; +, 10 - 25%; -, less than 10%. | | | | | |

As is clear from Table 2, the gelatin samples treated with pepsin, collagenase (C. histricum and one derived from the crab pancreas), chymotrypsin, elastase, bromelain, ficin and alkali protease had lower reactivity with the IgE in the allergic patients. However, excepting the sample treated with pepsin and elastase, these gelatin samples were not suitable for use in stabilizing trace proteins since their molecular weights were lower than the required value 3,500. The pepsin-treated gelatin had a weight average molecular weight of 7,600 well in excess of 3,500. The other enzymes tested were incapable of adequately reducing the reactivity of gelatin with IgE irrespective of whether the decomposed gelatin molecular weight was higher than 3,500 or not.

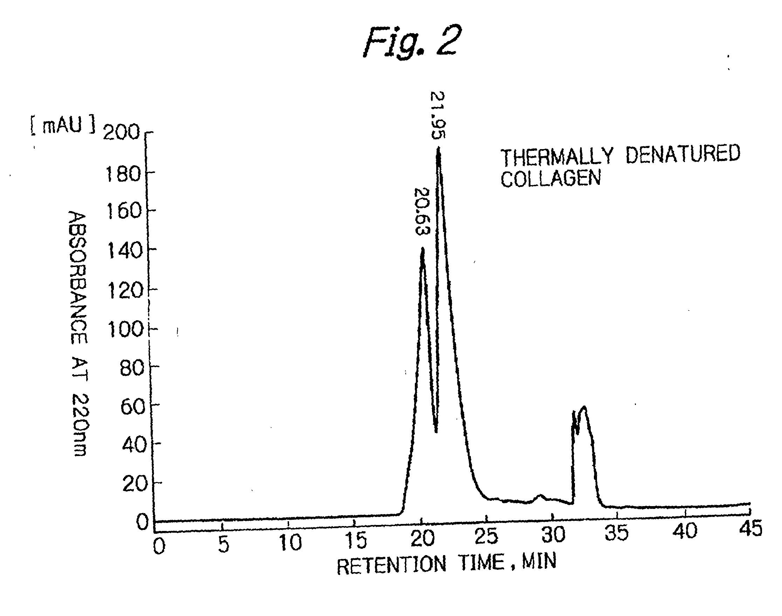

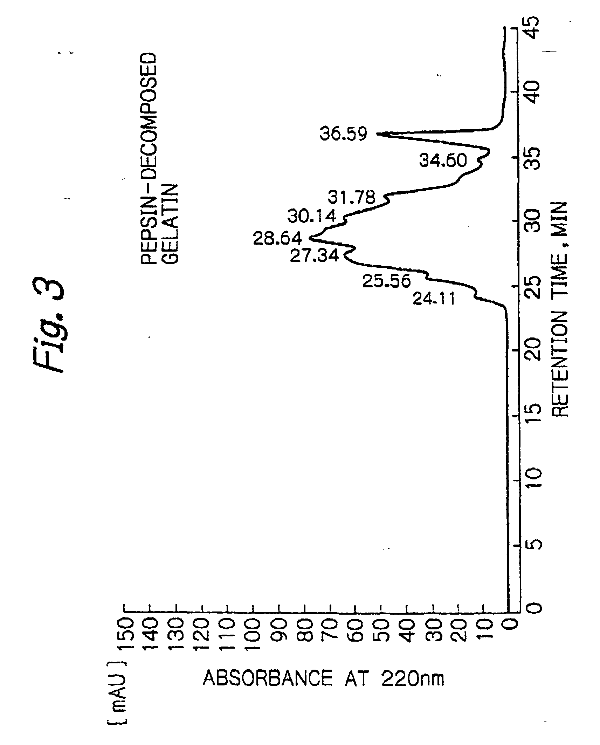

The untreated gelatin (i.e., thermally denatured SCE) and the samples of pepsin-decomposed gelatin were subjected to high-performance liquid chromatography and the separation patterns obtained are shown in Figs. 2 and 3. The band peak that occurred in the untreated gelatin at a retention time (RT) of 20.63 minutes was assigned to the gelatin chains having a molecular weight in excess of 200,000 and the peak for RT of 21.95 minutes was assigned to the gelatin chain that had a molecular weight of 100,000 (Fig. 2). The pepsin treated gelatin had peaks spread over a broad range of molecular weight for less than several tens of thousand. A maximum peak occurred at RT of 28.6 minutes and the calculated weight average molecular weight was 7,600 (Fig. 3). SDS-polyacrylamide gel electrophoresis:

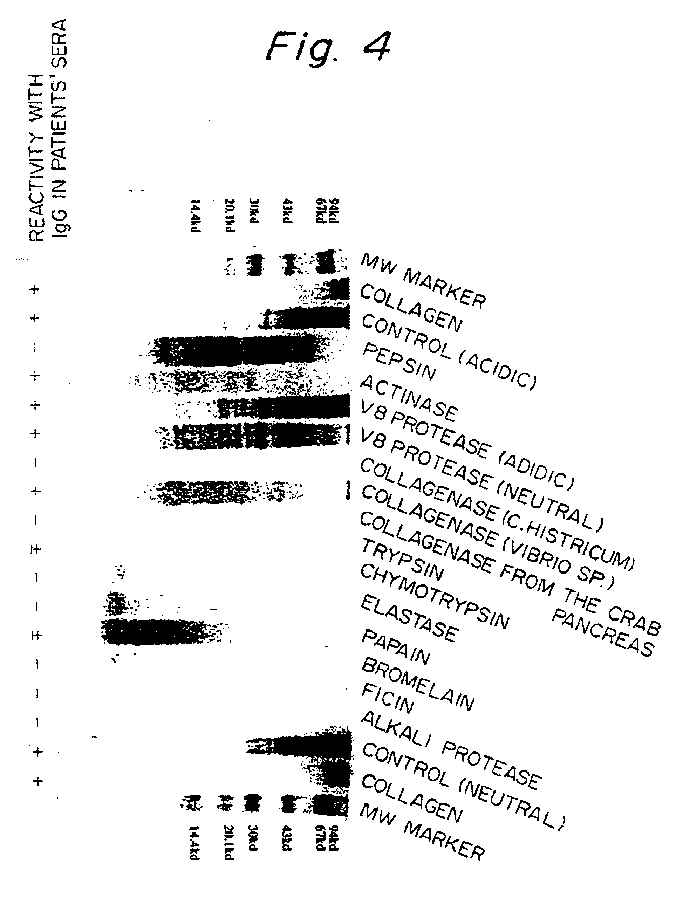

The enzyme-treated gelatin samples were electrophoresed in a 10% polyacrylamide gel and the resulting protein bands were rendered visible with Coomassie Brilliant Blue (see Fig. 4), in which the reactivity with IgE is indicated qualitatively under each band). As is clear from Fig. 4, the gelatin samples treated with collagenase (C. histricum and one derived from the crab pancreas), bromelain, ficin, alkali protease and other enzymes (except pepsin) that were lowered in reactivity with IgE had such small molecular weights that they slipped out the polyacrylamide gel and bands could scarcely be detected in electrophoresis. The gelatin samples treated with trypsin and papain were broken to such an extent that no bands could be detected and yet they maintained the reactivity with IgE. The gelatin samples decomposed with V8 protease and collagenase (Vibrio sp.) had high enough molecular weights to produce clearly visible bands in electrophoresis but their reactivity with IgE did not fall. The pepsin-treated gelatin showed a separation pattern comprising principal bands at 15 kd, 17 kd, 36 kd and 60 kd plus minor proportions of peptide bands with molecular weights in excess of 10,000.

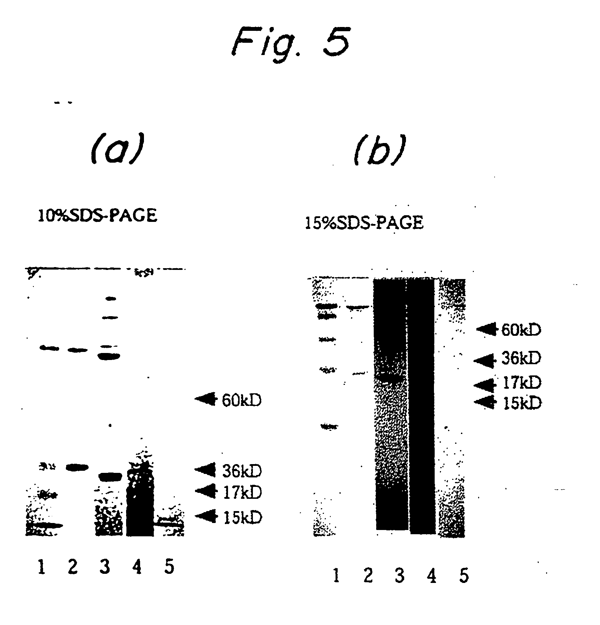

Other gelatin samples were treated with a tadpole-derived matrix metalloprotease which could sever collagen at only one site without causing loss of antigenicity and were later separated into two groups of peptides in a CM-cellulose column (Pharmacia); the first group consisted of the peptides derived from the α1 chain (α1TCa and α1TCb) and the second group consisted of the peptides derived from the α2 chain (α2TCa and α2TCb). These peptides were treated with pepsin (Sigma) at a substrate-to-enzyme weight ratio of 10 : 1 in 0.1M acetic acid at 37°C for 24 hours. Thereafter, the respective samples were analyzed by SDS-polyacrylamide gel eletrophoresis, with the gel concentration being varied at 10% and 15% (see Fig. 5). The results are shown in Table 5; lane 1 is a molecular weight marker showing, from top to bottom, bands for 103 kD, 77 kD, 48 kD, 34 kD, 28 kD and 20 kD; lane 2 is a fraction of collagen's α1 chain that was separated into 75 kD and 25 kD; lane 3 is a fraction of collagen's α2 chain that was separated into 75 kD and 25 kD; lane 4 is the pepsin-treated α1 chain of collagen; and lane 5 is the pepsin-treated α2 chain of collagen. Fig. 5 clearly shows that the separation pattern for the pepsin-treated gelatin having principal bands at 15 kd, 17 kd, 36 kd and 60 kd and a minor proportions of peptide bands with molecular weights in excess of 10,000 (see Fig. 4) are all derived from the α1 chain (lane 4 in Fig. 5). On the other hand, the antigenic α2 chain was completely decomposed with pepsin and no visible lanes were detected. (lane 5 in Fig. 5).

The pepsin-decomposed gelatin of the invention has a sufficient molecular weight to stabilize trace protein substances and yet it is a weak allergen. Therefore, it is useful as a stabilizer for medicines, food, cosmetics, notably for injectable trace protein substances such as vaccines.

## Claims

1. Use of a pepsin-treated low allergenic gelatin as a stabilizer for injectable trace protein substances wherein said gelatin has a weight average molecular weight of 3,500-20,000 as measured by gel permeation high-performance liquid chromatography and has a separation pattern having characteristic bands at 15 kd, 17 kd, 36 kd, and 60 kd in SDS-polyacrylamide gel electrophoresis.

2. An injection comprising an injectable trace protein substance, a stabilizer comprising a pepsin-treated low allergenic gelatin having a weight average molecular weight of 3,500-20,000 as measured by gel permeation high-performance liquid chromatography and having a separation pattern having characteristic bands at 15 kd, 17 kd, 36 kd, and 60 kd in SDS-polyacrylamide gel electrophoresis, and a pharmaceutically acceptable vehicle.

3. An injection according to claim 2, wherein said injectable trace protein substance comprises a vaccine and said vehicle comprises water.

## Patentansprüche

1. Verwendung von mit Pepsin behandelter, gering allergener Gelatine als Stabilisator für injizierbare Trace-Protein-Stoffe, wobei die Gelatine ein durchschnittliches Molekulargewicht von 3.500-20.000, gemessen durch Hochleistungsgelpermeationschromatographie, und ein Trennmuster mit charakteristischen Banden bei 15 kd, 17 kd, 36 kd und 60 kd in der SDS-Polyacrylamidgelelektrophorese hat.

2. Injektion, umfassend einen injizierbaren Trace-Protein-Stoff, einen Stabilisator, der mit Pepsin behandelte, gering allergene Gelatine umfasst, die ein durchschnittliches Molekulargewicht von 3.500-20.000, gemessen durch Hochleistungsgelpermeationschromatographie, und ein Trennmuster mit charakteristischen Banden bei 15 kd, 17 kd, 36 kd und 60 kd in der SDS-Polyacrylamidgelelektrophorese hat, und einen pharmazeutisch verträglichen Träger.

3. Injektion nach Anspruch 2, wobei der injizierbare Trace-Protein-Stoff einen Impfstoff umfasst und der Träger Wasser umfasst.

## Revendications

1. Utilisation d'une gélatine traitée à la pepsine faiblement allergénique en tant que stabilisateur pour substances injectables contenant des protéines marqueurs, **caractérisée en ce que** ladite gélatine a un poids moléculaire moyen de 3.500 à 20.000, tel que mesuré par chromatographie liquide de haute performance sur gel de perméation, et possède un profil de séparation ayant des bandes caractéristiques à 15 kD, 17 kD, 36 kD, et 60 kD en électrophorèse sur gel de SDS polyacrylamide.

2. Injection comprenant une substance injectable contenant des protéines marqueurs, un stabilisateur comprenant une gélatine traitée à la pepsine faiblement allergénique ayant un poids moléculaire moyen de 3.500 à 20.000, tel que mesuré par chromatographie liquide de haute performance sur gel de perméation, et possédant un profil de séparation ayant des bandes caractéristiques à 15 kD, 17 kD, 36 kD, et 60 Kd en électrophorèse sur gel de SDS -polyacrylamide, et un véhicule acceptable d'un point de vue pharmaceutique.

3. Injection selon la revendication 2, **caractérisée en ce que** la substance injectable contenant des protéines marqueurs comprend un vaccin, ledit véhicule comprenant de l'eau.
